(19) Europäisches Patentamt

European Patent Office

Office européen des brevets

(11) **EP 0 801 051 B1**

(12) **EUROPÄISCHE PATENTSCHRIFT**

(45) Veröffentlichungstag und Bekanntmachung des Hinweises auf die Patenterteilung:
**27.03.2002 Patentblatt 2002/13**

(51) Int Cl.$^7$: **C07C 68/00**, C07C 69/96

(21) Anmeldenummer: **97105177.6**

(22) Anmeldetag: **27.03.1997**

(54) **Verfahren zur kontinuierlichen Herstellung von Diarylcarbonaten**

Process for the continuous production of diaryl carbonates

Procédé de fabrication continue de carbonates de diaryle

(84) Benannte Vertragsstaaten:
**BE DE ES FR GB IT NL**

(30) Priorität: **09.04.1996 DE 19614062**

(43) Veröffentlichungstag der Anmeldung:
**15.10.1997 Patentblatt 1997/42**

(73) Patentinhaber: **BAYER AG**
**51368 Leverkusen (DE)**

(72) Erfinder:
• **Buysch, Hans-Josef, Dr.**
**47809 Krefeld (DE)**
• **Hesse, Carsten, Dr.**
**47800 Krefeld (DE)**
• **Rechner, Johann, Dr.**
**47906 Kempen (DE)**

(56) Entgegenhaltungen:
**EP-A- 0 614 876       EP-A- 0 736 325**

Bemerkungen:
Die Akte enthält technische Angaben, die nach dem Eingang der Anmeldung eingereicht wurden und die nicht in dieser Patentschrift enthalten sind.

**Beschreibung**

Die vorliegende Erfindung betrifft ein Verfahren zur Herstellung von Diarylcarbonaten durch Umsetzung einer aromatischen Hydroxyverbindung, beispielsweise Phenol, mit Kohlenmonoxid und Sauerstoff in Gegenwart eines Katalysators, eines Cokatalysators, eines quaternären Salzes und einer Base, worin man für die Reaktion einen stationär angeordneten oder in fluider Phase vorliegenden Träger-Katalysator einsetzt und die Reaktion in der kondensierten Phase durchführt, das Reaktionswasser kontinuierlich mit Reaktionsgas ausstrippt, die Reaktionslösung kontinuierlich austrägt, gebildetes Diarylcarbonat aus den Reaktionlösungen durch Extraktion, Kristallisation oder Destillation abtrennt, verlustfrei durch Kristallisation oder Destillation in hochreines Diarylcarbonat aufarbeitet und die restliche Reaktionslösung in den Reaktor zurückführt.

Es ist bekannt, organische Carbonate durch oxidative Umsetzung von aromatischen Hydroxyverbindungen mit Kohlenmonoxid in Gegenwart eines homogenen Edelmetall-Katalysators herzustellen (DE-OS 28 15 512). Als Edelmetall wird bevorzugt Palladium eingesetzt Zusätzlich können ein Cokatalysator, beispielsweise Mangan- oder Kobaltsalze, eine Base, ein quaternäres Salz, verschiedene Chinone bzw. Hydrochinone und Trockenmittel eingesetzt werden. Dabei kann in einem Lösungsmittel, bevorzugt Methylenchlorid, gearbeitet werden. Die Übertragung dieser Reaktion in ein industrielles Verfahren gestaltet sich jedoch schwierig, da ein homogener Edelmetall-Katalysator einsetzt wird, der aufwendig zurückgewonnen werden muß, da das Edelmetall einen erheblichen Kostenfaktor darstellt. Verluste an Edelmetall-Katalysator müssen kostenintensiv ersetzt werden, außerdem dürfen keine Reste des Edelmetall-Katalysators im Produkt verbleiben. Für Katalysatorsysteme, die zur oxidativen Carbonylierung von aromatischen Hydroxyverbindungen zu Diarylcarbonaten verwendet werden, ist eine wirtschaftliche und effiziente Rückgewinnung bisher jedoch nicht bekannt.

In JP 04/257 546 und JP 04/261 142 wird in je einem Beispiel ein Träger-Katalysator beschrieben, bei dem Siliciumcarbid-Granulat als Material für einen Träger-Katalysator in einer Destillationskolonne verwendet wird. Obwohl in den betreffenden Beispielen unter drastischen Bedingungen (hoher Druck, hohe Temperatur) gearbeitet wird, ermöglicht dieser Katalysator nur sehr geringe Raum-Zeit-Ausbeuten, was eine ökonomische Herstellung von aromatischen Carbonaten mit derartigen Träger-Katalysatoren unmöglich macht. Weiterhin wird in JP 04/257 546 das kontinuierliche Abdestillieren des Reaktionswassers in der Destillationskolonne beschrieben; die übrigen Reaktionsprodukte werden gemeinsam mit dem Edelmetall-Katalysator im Destillationssumpf entnommen. Nachteilig bei diesem Verfahren ist, daß man zur Entfernung des Reaktionswassers in einer Destillationskolonne arbeiten muß, die aufgrund ihrer Konstruktion nur kurze Verweilzeiten ermöglicht und nur zur Entfernung flüchtiger Komponenten geeignet ist. Dem Fachmann ist außerdem bekannt, daß unter Destillationsbedingungen die Löslichkeit von Gasen, hier Reaktionsgas $CO/O_2$, stark zurückgeht. Eine in üblicher Weise betriebene Destillationskolonne besitzt einen starken Temperaturgradienten entlang der Vertikalen, dessen Aufrechterhaltung sehr hohe Sumpftemperaturen erfordert. Unter diesen Bedingungen wird das im Sumpf angereicherte Katalysatorsystem erheblich geschädigt, was zu Katalysatorverlusten, Diarylcarbonatzersetzung und weiteren Nebenreaktionen führt. Die mit diesem Verfahren realisierbaren Raum-Zeit-Ausbeuten sind demzufolge mit nur 17,8 g/l×h sehr niedrig. Destillationskolonnen sind daher als Reaktoren für die oxidative Carbonylierung von aromatischen Hydroxyverbindungen zu Diarylcarbonaten nicht geeignet. Ein weiterer Nachteil besteht im extrem hohen Katalysatoraufwand, der für diese Raum-Zeit-Ausbeuten notwendig ist. So werden im Beispiel 1 von JP 04/257 546 bei einer Belastung von 182 g Phenol pro Stunde insgesamt 3 g/h Palladiumverbindung und 14,4 g/h quaternäres Ammoniumsalz eingesetzt. Bei einer angegebenen Ausbeute von 35 g Diphenylcarbonat pro Stunde werden also in einer Stunde nur 16,3 Katalysezyklen erzielt. Dies bedeutet, daß zur Herstellung eines Kilogramms Diphenylcarbonat nach diesem Verfahren mindestens 30 g reines Palladium (entsprechend ~90 g Palladiumverbindung) benötigt werden. Dies bedingt sehr hohe Investitionskosten für den Katalysator. Das Problem der Abtrennung und Wiedergewinnung des Edelmetall-Katalysators aus den Reaktionsprodukten ist nicht gelöst, so daß eine wirtschaftliche Nutzung des Verfahrens unmöglich ist. Die Verwendung großer Mengen Halogenide bei hohen Temperaturen von 150 bis 180°C, wie sie in diesem Verfahren erforderlich sind, führt zu großen Korrosionsproblemen, die einen hohen apparativen Aufwand bedingen. Dem Fachmann ist außerdem bekannt, daß unter den angegebenen Reaktionsbedingungen das bevorzugt als Salz eingesetzte Iodid des quaternären Salzes nicht stabil ist und in erheblichem Maße zu Iod oxidiert wird. Dies führt zu großen Verlusten des quaternären Salzes und zur Bildung von Nebenprodukten, was die Selektivität und damit die Wirtschaftlichkeit dieses Verfahrens zusätzlich stark beeinträchtigt.

In JP 04/261 142 wird ein Verfahren beschrieben, bei dem wie in JP 04/257 546 gearbeitet wird, jedoch mit dem Unterschied, daß an die Destillationskolonne zur Verweilzeiterhöhung zusätzliche Reaktoren angebracht wurden. Die oben angeführten Probleme von Korrosion, Katalysatoraufwand und Verlust an quaternärem Salz sowie den damit verbundenen Nebenreaktionen sind auch in dieser Anmeldung nicht gelöst. Die vorgeschlagene Apparatur bringt ebenfalls keine Vorteile, da alle Nachteile, die durch Verwendung von Destillationskolonnen als Reaktoren entstehen, weiterhin auftreten. Zwar wird die Verweilzeit durch die zusätzlichen Reaktoren vergrößert, die vorgeschlagene Konstruktion führt aber zu einer erheblichen Rückvermischung innerhalb der Apparatur, so daß Nebenreaktionen in verstärktem Maße ablaufen können, wodurch die Selektivität zurückgeht. So wird im Ausführungsbeispiel 1 von JP 04/261 142 eine Selektivität von nur 97% gegenüber 99% im vergleichbaren Beispiel 1 von JP 04/257 546 erzielt. Die mit diesem Verfahren erzielbaren Raum-Zeit-Ausbeuten sind mit ca. 9 g/l×h noch geringer als bei JP 04/257 546. Durch die zusätzlich angebrachten Reaktoren wird eine effektive Entfernung des Reaktionswassers unmöglich. Bei der vorgeschlagenen Verfahrensweise wird das während der Reaktion in den Reaktoren gebildete Reaktionswasser nämlich erst nachträglich in der Destillationskolonne entfernt. Unter den Reaktionsbedingungen wird in den Reaktoren gebildetes Carbonat hydrolytisch wieder gespalten, wodurch nur sehr niedrige Umsätze erzielbar sind.

In EP A1 0 614 876 wird ein Verfahren zur Herstellung von Diarylcarbonaten in diskontinuierlicher Verfahrensweise unter Verwendung eines heterogenen Katalysator-systems aus Pol und Cer beschrieben.

In EP A1 0 667 336 wird ein kontinuierliches, Verfahren zur Hertellung von Diarylcarbonaten beschrieben, in welchem homogene Katalysatoren eingesetzt werden.

Es ist also bisher kein Verfahren zur Herstellung von Diarylcarbonaten durch Umsetzung einer aromatischen Hydroxyverbindung mit Kohlenmonoxid und Sauerstoff bekannt, das eine wirtschaftliche, kontinuierliche Prozessführung mit hoher Raum-Zeit-Ausbeute ohne Katalysatorschädigung erlaubt. Es bestand daher die Aufgabe, ein Verfahren mit hoher Aktivität und Selektivität zu finden, das die wirtschaftliche, kontinuierliche Herstellung von Diarylcarbonaten durch Umsetzung einer aromatischen Hydroxyverbindung mit Kohlenmonoxid und Sauerstoff ohne Katalysatorschädigung erlaubt.

Es wurde nun überraschend gefunden, daß die beschriebenen Nachteile überwunden werden können, wenn man die Reaktion in Gegenwart eines stationär angeordneten oder in fluider Phase vorliegenden Trägerkatalysators in der kondensierten Phase durchführt, die Reaktionslösung kontinuierlich aus dem Reaktor entnimmt, gebildetes Diarylcarbonat durch Kristallisation, Destillation oder Extraktion aus der Reaktionslösung abtrennt, durch weitere Kristallisation oder Destillation weitgehend verlustfrei in hochreines Diarylcarbonat aufarbeitet und die restliche Reaktionslösung in den Reaktor zurückführt.

Die Erfindung betrifft demnach ein Verfahren zur kontinuierlichen Herstellung von Diarylcarbonaten der Formel

$$R^1\text{-O-CO-O-}R^1 \qquad (I)$$

durch Umsetzung von aromatischen Hydroxyverbindungen der Formel

$$R^1\text{-OH} \qquad (II),$$

wobei in den Formeln

$R^1$ nicht substituiertes oder substituiertes $C_6$-$C_{15}$-Aryl, bevorzugt nicht substituiertes oder substituiertes Phenyl, besonders bevorzugt nicht substituiertes Phenyl bedeutet,

mit Kohlenmonoxid und Sauerstoff bei 30-200°C, bevorzugt 30-150°C, besonders bevorzugt 40-120°C und 1-120 bar, bevorzugt 2-80 bar, besonders bevorzugt 5-25 bar, in Gegenwart eines Platingruppenmetalls oder einer Verbindung eines Platingruppenmetalls als Katalysator, eines Cokatalysators, eines quaternären Salzes und einer Base, bei dem der Katalysator gemeisam mit dem Cokatalysator als stationär angeordneter oder in fluider Phase vorliegender Trägerkatalysator eingesetzt und die Reaktion in der kondensierten Phase durchgeführt wird.

Am Beispiel der Bildung von Diphenylcarbonat kann das erfindungsgemäße Verfahren wie folgt formelmäßig dargestellt werden:

$$2\ C_6H_5\text{-OH} + CO + \tfrac{1}{2}\ O_2 \rightarrow (C_6H_5O)_2CO + H_2O.$$

Erfindungsgemäß einsetzbare und den Diarylcarbonaten zugrundeliegende aromatische Hydroxyverbindungen sind beispielsweise Phenol, o-, m- oder p-Kresol, o-, m- oder p-Chlorphenol, o-, m- oder p-Ethylphenol, o-, m- oder p-Propylphenol, o-, m- oder p-Methoxyphenol, 2,6-Dimethylphenol, 2,4-Dimethylphenol, 3,4-Dimethylphenol, 1-Naphthol, 2-Naphthol, o-, m-, p-Phenylphenol und Bisphenol A, bevorzugt Phenol. Allgemein handelt es sich im Falle einer Substitution der aromatischen Hydroxyverbindung um 1 oder 2 Substituenten in der Bedeutung von $C_1$-$C_4$-Alkyl, $C_1$-$C_4$-Alkoxy, Fluor, Chlor oder Brom oder um den Rest HO-$C_6H_4$-C(CH$_3$)$_2$-.

Die für das erfindungsgemäße Verfahren einsetzbaren Trägerkatalysatoren enthalten im reaktionsbereiten Zustand ein Platingruppenmetall oder eine Verbindung eines Platingruppenmetalls, bevorzugt ein Platingruppenmetall-Halogenid oder eine Platingruppenmetall-Halogenid enthaltende Komplexverbindung oder eine Verbindung, die unter Reaktionsbedingungen in ein Platingruppenmetall, ein Platingruppenmetall-Halogenid oder eine Platingruppenmetall-Halogenid enthaltende Komplexverbindung übergeführt werden kann, in einer Menge von 0,01 - 15 Gew.-%, bevorzugt 0,05 - 10 Gew.-%, gerechnet als Platingruppenmetall und bezogen auf das Gesamtgewicht des Katalysators. Von den Platingruppenmetallen kommen Pt, Ir, Pd, Ru oder Rh oder mehrere von ihnen in Betracht. Pd und Rh oder ein Gemisch von ihnen sind jedoch bevorzugt, insbesondere Pd. Zusätzlich ist eine cokatalytisch wirkende Metallverbindung aus der Gruppe Mn, Cu, Co, V, Zn, und Mo in einer Menge von 0,01 - 15 Gew.-%, bevorzugt 0,05 - 10 Gew.-%, gerechnet als Metall und bezogen auf das Gesamtgewicht des Katalysators, enthalten. Solche Katalysatoren liegen als vollständig heterogenes katalytisches System vor und vermeiden so die Vermischung und Verunreinigung des Reaktionsproduktes mit Katalysatoranteilen.

Als Katalysatorträger eignen sich alle technisch üblichen Katalysatorträger auf der Basis von Kohle, Elementoxiden, Elementcarbiden oder Elementsalzen in verschiedenen Anwendungsformen. Beispiele für kohlenstoffhaltige Träger sind Koks, Graphit, Ruß oder Aktivkohle. Beispiele für Elementoxid-Katalysatorträger sind $SiO_2$ (natürliche oder synthetische Kieselsäuren, Quarz), $Al_2O_3$ ($\alpha$-, $\gamma$-$Al_2O_3$), Tonerden, natürliche und synthetische Alumosilicate (Zeolithe), $TiO_2$ (Rutil, Anatas), $ZrO_2$ oder ZnO. Beispiele für Elementcarbide und -salze sind SiC, $AlPO_4$, $BaSO_4$, $CaCO_3$ u.a. Sie können sowohl im Sinne chemisch einheitlicher Reinsubstanzen als auch im Gemisch eingesetzt werden. Für die erfindungsgemäße Verwendung als Katalysatorträger eignen sich sowohl stückige als auch pulverförmige Materialien. Für den Fall der Anordnung des Trägerkatalysators als Festbett wird der Träger vorzugsweise als Formkörper, beispielsweise in Form von Kugeln, Zylindern, Stäbchen, Hohlzylindern, Ringen, usw. eingesetzt. Wahlweise können Katalysatorträger weiter durch Extrudieren, Tablettieren, gegebenenfalls unter Zumischen weiterer Katalysatorträger oder Bindemitteln, wie $SiO_2$ oder $Al_2O_3$, und Calcinieren modifiziert werden. Darstellung und Weiterverarbeitung der erfindungsgemäß verwendeten Katalysatorträger sind dem Fachmann wohl bekannt und Stand der Technik. Edelmetalle können nach bekannten Methoden, beispielsweise durch Tränken, Fällen oder Adsorption, aufgebracht werden.

Geeignet als quaternäres Salz für den Einsatz im erfindungsgemäßen Verfahren sind Ammonium- und Phosphoniumsalze, die als organische Reste gleiche oder unterschiedliche $C_6$- bis $C_{10}$-Aryl-, $C_7$- bis $C_{12}$-Aralkyl- und/oder $C_1$- bis $C_{20}$-Alkyl-Reste und als Anion ein Halogenid, Tetrafluoroborat oder Hexafluorophosphat tragen oder ein Gemisch mehrerer von ihnen. Bevorzugt werden Ammoniumsalze, die als organische Reste $C_6$- bis $C_{10}$-Aryl-, $C_7$- bis $C_{12}$-Aralkyl- und/oder $C_1$- bis $C_{20}$-Alkyl-Reste und als Anion ein Halogenid tragen, eingesetzt, besonders bevorzugt ist Tetrabutylammoniumbromid. Die Menge eines solchen quaternären Salzes beträgt 0,1 bis 50 Gew.-%, bezogen auf das Gewicht des Reaktionsgemisches. Vorzugsweise beträgt diese Menge 0,5 bis 15 Gew.-%, besonders bevorzugt 1 bis 5 Gew.-%.

Für das erfindungsgemäße Verfahren können beliebige sowohl organische als auch anorganische Basen oder Mischungen derselben verwendet werden. Als Beispiele für anorganische Basen seien, ohne das erfindungsgemäße Verfahren einzuschränken, Alkalimetallhydroxide und -carbonate, -$C_2$-$C_{12}$-carboxylate oder andere Salze schwacher Säuren sowie Alkalisalze von aromatischen Hydroxyverbindungen der Formel (II), z. B. Alkalimetallphenolate, genannt. Selbstverständlich können in das erfindungsgemäße Verfahren auch die Hydrate von Alkalimetallphenolaten eingesetzt werden. Als Beispiel für ein solches Hydrat sei hier, ohne das erfindungsgemäße Verfahren einzuschränken, Natriumphenolat-trihydrat genannt. Die Menge an zugesetztem Wasser ist jedoch vorzugsweise so bemessen, daß pro Mol Base maximal 5 Mol Wasser eingesetzt werden. Höhere Wasserkonzentrationen führen meist zu schlechteren Umsätzen und zur Zersetzung gebildeter Carbonate. Als organische Basen seien, ohne das erfindungsgemäße Verfahren einzuschränken, tertiäre Amine, die als organische Reste gleiche oder verschiedene von $C_6$- bis $C_{10}$-Aryl-, $C_7$- bis $C_{12}$-Aralkyl- und/ oder $C_1$- bis $C_{20}$-Alkyl-Reste tragen können, sowie Pyridinbasen oder hydrierte Pyridinbasen, genannt, beispielsweise Triethylamin, Tripropylamin, Tributylamin, Trioctylamin, Benzyldimethylamin, Dioctylbenzylamin, Dimethylphenethylamin, 1-Dimethylamino-2-phenylpropan, Pyridin, N-Methylpiperidin, 1,2,2,6,6-Pentamethylpiperidin. Bevorzugt wird als Base ein Alkalisalz einer aromatischen Hydroxyverbindung verwendet, besonders bevorzugt ein Alkalisalz der aromatischen Hydroxyverbindung, die auch zum organischen Carbonat umgesetzt werden soll. Diese Alkalisalze können Lithium-, Natrium-, Kalium-, Rubidium- oder Cäsiumsalze sein. Bevorzugt werden Lithium-, Natrium- und Kaliumphenolat, besonders bevorzugt Natriumphenolat, eingesetzt. Die Menge der Base beträgt 0,01 bis 20 Gew.-%, bezogen auf das Gewicht des Reaktionsgemisches. Vorzugsweise beträgt diese Menge 0,05 bis 15 Gew.-%, besonders bevorzugt 0,1 bis 5 Gew.-%.

Die Base kann dem Reaktionsgemisch als reine Verbindung in fester Form oder als Schmelze zugesetzt werden. In einer weiteren Ausführungsform der Erfindung wird die Base dem Reaktionsgemisch als Lösung, die 0,1 bis 80 Gew.-%, bevorzugt 0,5 bis 65 Gew.-%, besonders bevorzugt 1 bis 50 Gew.-% der Base enthält, zugesetzt. Als Lösungsmittel können hierbei sowohl Alkohole oder Phenole, wie z.B. das umzusetzende Phenol, als auch inerte Lösungsmittel verwendet werden.

Als Beispiele für Lösungsmittel seien Dimethylacetamid, N-Methylpyrrolidinon, Dioxan, t-Butanol, Cumylalkohol, Isoamylalkohol, Tetramethylharnstoff, Diethylenglykol, halogenierte Kohlenwasserstoffe (z.B. Chlorbenzol oder Dichlorbenzol) und Ether genannt. Diese Lösungsmittel können allein oder in beliebiger Kombination miteinander eingesetzt werden. So besteht eine Ausführungsform des erfindungsgemäßen Verfahrens beispielsweise darin, daß man die Base in einer Phenolschmelze löst, die mit einem Lösungsmittel verdünnt wurde. Bevorzugt wird die Base in der Schmelze einer aromatischen Hydroxyverbindung gelöst, besonders bevorzugt in einer Schmelze der aromatischen Hydroxyverbindung, die zum organischen Carbonat umgesetzt werden soll. Ganz besonders bevorzugt wird die Base, in Phenol gelöst, zugesetzt; dies bedeutet in bevorzugter Weise den Verzicht auf weitere Lösungsmittel der genannten Art.

Das erfindungsgemäße Verfahren wird bei 30 bis 200°C, bevorzugt bei 30 bis 150°C, besonders bevorzugt bei 40 bis 120°C und bei einem Druck von 1 bis 120 bar, bevorzugt von 2 bis 80 bar, besonders bevorzugt von 5 bis 25 bar, durchgeführt.

Das erfindungsgemäße Verfahren wird vorzugsweise ohne Lösungsmittel durchgeführt, d.h. lediglich in der Schmelze der umzusetzenden aromatischen Hydroxyverbindung. Selbstverständlich können auch inerte Lösungsmittel verwendet werden, wie sie als Beispiele zum Lösen der Base oben genannt sind.

Das Reaktionsgas für das erfindungsgemäße Verfahren besteht aus Kohlenmonoxid (CO) und Sauerstoff ($O_2$). Zusätzlich kann ein Inertgas enthalten sein. Das Reaktionsgas wird in einer Menge von 1 bis 100 000 Nl pro Liter Reaktionslösung, bevorzugt 5 bis 50 000 Nl pro Liter Reaktionslösung und besonders bevorzugt 10 bis 10 000 Nl pro Liter Reaktionslösung eingeleitet.

Die Zusammensetzung der Bestandteile CO und $O_2$ kann in weiten Konzentrationsgrenzen variiert werden, es wird jedoch zweckmäßigerweise ein CO:$O_2$-Molverhältnis (normiert auf CO) von 1:(0,001-1,0) bevorzugt 1:(0,01-0,5) und besonders bevorzugt von 1:(0,02-0,3) eingestellt. Der $O_2$-Partialdruck ist bei diesen Molverhältnissen groß genug, um hohe Raum-Zeit-Ausbeuten erreichen zu können und gleichzeitig keine explosionsfähigen CO/$O_2$-Gasgemische bilden zu können. Die Bestandteile unterliegen keinen besonderen Reinheitsanforderungen, so kann Synthesegas als CO-Quelle und Luft als $O_2$-Träger dienen, es ist jedoch darauf zu achten, daß keine Katalysatorgifte, wie z.B. Schwefel oder dessen Verbindungen, eingetragen werden. In der bevorzugten Ausführungsform des erfindungsgemäßen Verfahrens werden reines CO und reiner $O_2$ verwendet In einer weiteren bevorzugten Ausführungsform des erfindungsgemäßen Verfahrens werden CO und $O_2$ unabhängig voneinander dosiert. Die Dosierung des $O_2$ kann hierbei gegebenenfalls zusammen mit Inertgas erfolgen. Im Falle der Verwendung einer Reaktorkaskade anstelle eines Einzelreaktors erfolgt die separate $O_2$-Dosierung bevorzugt so, daß in jedem der Reaktoren die optimale $O_2$-Konzentration gewährleistet ist. Inerte Bestandteile der Reaktionsgase im erfindungsgemäßen Verfahren können Stickstoff, Wasserstoff, Edelgase sowie unter Reaktionsbedingungen beständige organische Verbindungen, die bevorzugt mit Wasser ein Azeotrop bilden, sein. Die Konzentration an Inertgas im Reaktionsgas kann 0-30 Vol.-%, bevorzugt 0-15 Vol.-%, besonders bevorzugt 0-5 Vol.-%, betragen. Die Konzentration 0 Vol.-% stellt den Spezialfall des bevorzugten inertgasfreien Zustands dar.

Durch ein im Abgasstrom befindliches Trennorgan, wie z.B. Dephlegmator, Destillationskolonnen mit Böden oder Packung und weitere dem Fachmann bekannte Apparate, kann der größte Teil des mitgerissenen Phenols bzw. Lösungsmittels wieder in den Reaktor zurückgeführt werden. Das mit Wasser angereicherte überschüssige Reaktionsgas wird in der bevorzugten Ausführungsform nach der Wasserabtrennung wieder in den Reaktor zurückgeführt. Die Wasserabtrennung aus dem Reaktionsgas erfolgt mit bekannten Mitteln, z.B. durch Adsorption, Absorption oder bevorzugt durch Abkühlung des unter Druck stehenden Gases und Kondensation des Wassers. (Ullmann's Encyclopedia of Industrial Chemistry, 5. Auflage, Volume A5, Seiten 203ff, Weinheim 1986; Ullmann's Encyclopedia of Industrial Chemistry, 5. Auflage, Volume A12, Seiten 169 ff, 1989).

Als Reaktoren für das erfindungsgemäße Verfahren kommen im Gegensatz zu Destillationskolonnen solche in Betracht, in denen alle Reaktionsteilnehmer mit Ausnahme der permanenten Gase CO und $O_2$ in kondensierter Phase vorliegen. Der Katalysator kann hierbei stationär angeordnet sein oder in fluider Phase vorliegen. Beispiele solcher Reaktoren für Katalysatoren, die in fluider Phase vorliegen, sind Rührkessel, Fluidized-bed-Reaktoren und Blasensäulen, wobei diese als Einzelreaktoren oder als Kaskade eingesetzt werden können. In einer Kaskade können 2 bis 15, bevorzugt 2 bis 10, besonders bevorzugt 2 bis 5 Reaktoren hintereinandergeschaltet sein.

Wird der Trägerkatalysator als Pulver (für die fluide Phase) eingesetzt, sind zur Vermischung der Reaktionskomponenten die zu verwendenden Rührbehälter mit dafür brauchbaren Rührern ausgestattet; in Blasensäulen und Fluidized-bed-Reaktoren wird das Reaktionsgemisch durch die Gase CO und $O_2$ vermischt. Beim Arbeiten mit Trägerkatalysator-Pulvern als Suspension in Rührgefäßen oder Blasensäulen werden Mengen von 0,001 bis 50 Gew.-%, bevorzugt von 0,01 bis 20 Gew.-%, besonders bevorzugt von 0,1 bis 10 Gew.-% Träger-Katalysator-Pulver, bezogen auf die eingesetzte Menge an aromatischer Hydroxyverbindung, verwendet.

Suspendierte Katalysatoren können aus der Reaktionsmischung z.B. durch Filtration, Sedimentation oder Zentrifugieren abgetrennt und in den Reaktor zurückgeführt werden. Selbstverständlich können flüssige Teile der Reaktionsmischung auch über eine Fritte dem Reaktor entnommen werden, wobei der Katalysator im Reaktor verbleibt.

In bevorzugten Ausführungsformen wird der heterogene Trägerkatalysator als Formkörper stationär in Rührkesseln, Blasensäulen, Festbettreaktoren, Rieselphasen-Reaktoren oder Kaskaden dieser Reaktoren, wobei die verschiedenen Reaktortypen auch gleichzeitig in einer Kaskade vorkommen können, angeordnet; derartige Reaktoren sind beschrieben in Ullmann's Encyclopedia of Industrial Chemistry, 5. Auflage 1989, Volume B4 Part B, Seiten 98 ff. Im Falle einer kontinuierlichen Fahrweise, wobei Gas- und Flüssigphase im Gegen- oder Gleichstrom geführt werden können, sowie in der Rieselphase am Festbett-Katalysator werden Belastungen von 0,01 bis 20 g, bevorzugt 0,05 bis 10 g, besonders bevorzugt 0,1 bis 5 g an aromatischer Hydroxyverbindung pro Gramm Trägerkatalysator und Stunde eingestellt. Die stationär angeordneten Trägerkatalysatoren können über lange Zeit im Reaktor verbleiben.

Als Blasensäulen und analoge Reaktoren können in dem erfindungsgemäßen Verfahren folgende Typen eingesetzt werden: einfache Blasensäulen; Blasensäulen mit Einbauten, wie z.B.: Blasensäulen mit parallelen Kammern, Kaskaden-Blasensäulen mit Siebböden oder Einlochböden, Blasensäulen mit Packungen, mit statischen Mischern, pulsierende Siebbodenblasensäulen; Schlaufenreaktoren wie z.B.: Mammutschlaufenreaktoren, Abstromschlaufenreaktoren, Strahlschlaufenreaktoren, Freistrahlreaktoren, Strahldüsenreaktoren; Blasensäulen mit Flüssigkeitstauchstrahler, Abstrom-Aufstrom-Blasensäulen und weitere dem Fachmann bekannte Blasensäulenreaktoren (H.Gerstenberg, Chem. Ing. Tech. 61 (1979) Nr. 3, S. 208-216; W.-D. Deckwer, Reaktionstechnik in Blasensäulen, Otto Salle Verlag 1985). In der bevorzugten Ausführungsform kommen Blasensäulenreaktoren und Blasensäulen-Kaskaden zum Einsatz, die eine effektive Vermischung von Gas- und Flüssigphase erlauben, wie beispielsweise Kaskaden-Blasensäulen und Schlaufenreaktoren. Zur Aufrechterhaltung einer guten Durchmischung von Flüssigkeit und Reaktionsgas können Verteilungs- und Redispergierorgane entlang der Längsachse der Blasensäulenreaktoren angebracht sein. Als feste Redispergierorgane kommen Einlochböden, Lochplatten, Siebböden sowie weitere dem Fachmann bekannte Einbauten zum Einsatz. Derartige Reaktoren sind bekannt und werden beispielsweise in Catal.Rev.-Sci.-Eng. (1995), Vol 37(2), S.227-309 beschrieben. Für die Erstdispergierung des Reaktionsgases in der flüssigen Phase bei der Eindosierung sind übliche Vorrichtungen wie poröse Sinterplatten, Lochplatten, Siebböden, Einsteckrohre, Düsen, Begasungsringe und weitere dem Fachmann bekannte Dispergiervorrichtungen einsetzbar.

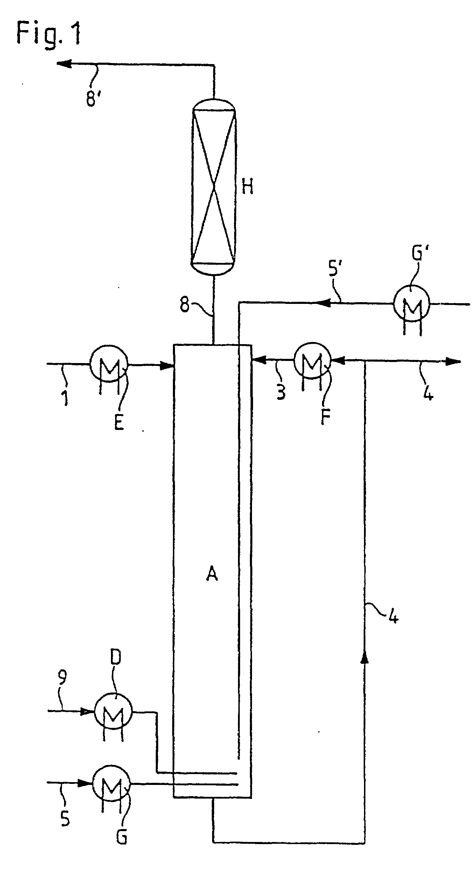

In den bevorzugten Ausführungsformen des erfindungsgemäßen Verfahrens kommt eine kontinuierliche Arbeitsweise im Einzelreaktor oder in einer Kaskade aus Reaktoren zum Einsatz. In den Figuren 1, 2 und 3 sind beispielhaft Arbeitsweisen mit einem bzw. drei Reaktoren (A, B und C) dargestellt, wobei die erfindungsgemäße Arbeitsweise nicht auf diese Beispiele eingeschränkt werden soll.

Eine beispielhafte Ausführungsform des erfindungsgemäßen Verfahrens besteht darin, daß man die Lösung der Komponenten des Reaktionssystems (aromatische Hydroxyverbindung, Base, quaternäres Salz und ggf. Cokatalysator) in den oben angegebenen Konzentationen kontinuierlich über Leitung (1), Fig. 1, dem Reaktor (A) zusetzt. Durch ein vorgeschaltetes Erhitzerelement (E) können die flüssigen Reaktionskomponenten gegebenenfalls auf die vorgesehene Reaktionstemperatur vorgeheizt werden. Die aus (A) zu entnehmende Flüssigphase wird am unteren Reaktorende entnommen und über die Leitung (4) der Aufarbeitung zugeführt. Die Regelung des gewünschten Füllstandes im kontinuierlich betriebenen Reaktor erfolgt nach bekannten Methoden. Gegebenenfalls kann ein Teilstrom über Leitung (3) wieder nach (A) zurückgeführt werden. Durch ein vorgeschaltetes Erhitzerelement (F) können die flüssigen Reaktionskomponenten gegebenenfalls wieder auf die vorgesehene Reaktionstemperatur vorgeheizt werden. Das aus CO und $O_2$ bestehende Reaktionsgas kann über die Leitung (5) oder (5') am unteren Ende von (A) eingeleitet und gegebenenfalls zuvor durch einen Vorerhitzer (G) bzw. (G') auf die Reaktionstemperatur vorgeheizt werden. Die $O_2$-Dosierung kann hierbei unabhängig von CO und Inertgas bzw. zusammen mit Inertgas erfolgen. Im Falle einer separaten $O_2$-Dosierung erfolgt diese durch Leitung (9) und Vorerhitzer (D). Dabei werden CO und $O_2$, in den oben angegebenen Mengen, entweder, im Falle eines Rührkessels, durch einen Begasungsrührer oder andere bekannte Gasverteilungsorgane in der Reaktionsmischung verteilt. Das überschüssige Reaktionsgas verläßt zusammen mit dem Reaktionswasser und mitgerissenem Edukt (II) über Leitung (8) den Reaktor. Im Trennorgan (H) wird der größte Teil des Edukts (II) abgetrennt und in den Reaktor (A) zurückgeführt. Über Leitung (8') verläßt das überschüssige Reaktionsgas zusammen mit dem Reaktionswasser den Reaktor. Das Reaktionswasser wird aus dem Reaktionsgas in bekannter Weise entfernt. Das Reaktionsgas wird anschließend zusammen mit dem Ersatz des verbrauchten Reaktionsgases dem Reaktor (A) wieder zugeführt.

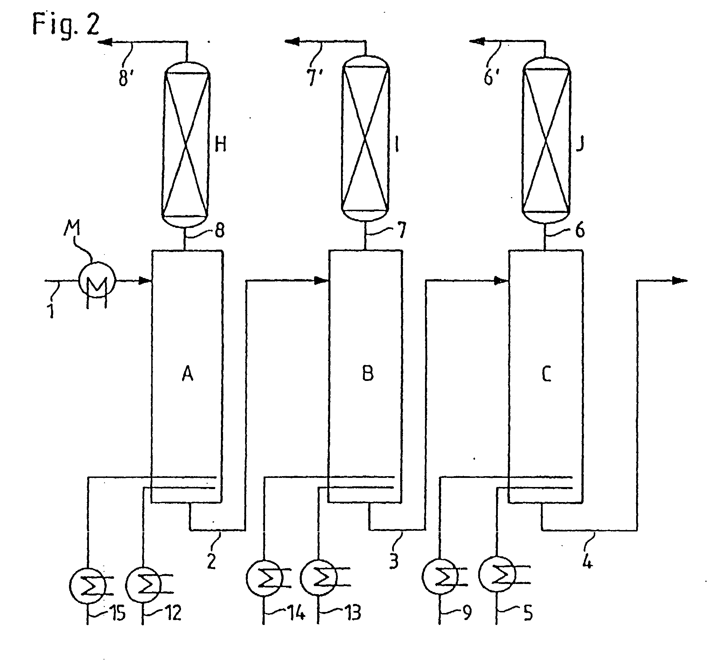

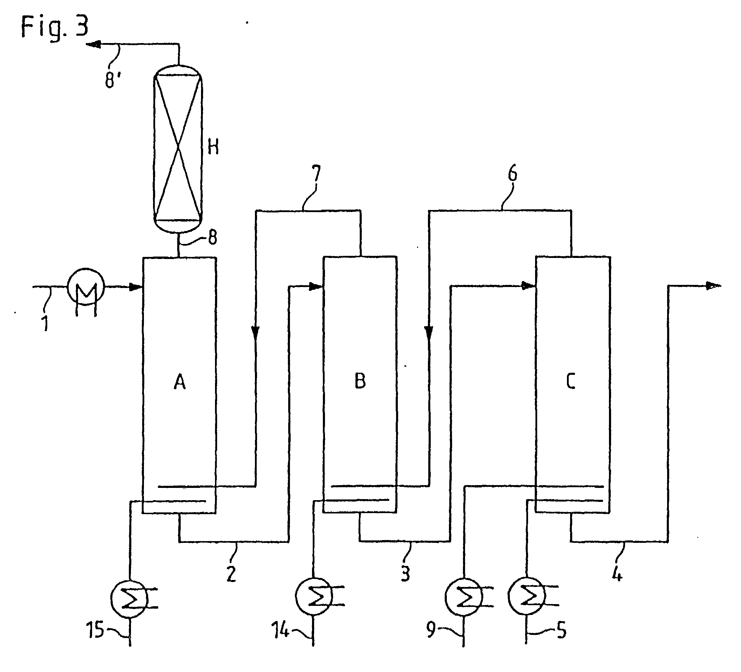

Im Falle der Verwendung einer Reaktorkaskade (Fig. 2 bzw. 3) werden die oben beschriebenen flüssigen Reaktionskomponenten in den ersten Reaktor (A) eindosiert und können gegebenenfalls in einem vorgeschalteten Erhitzerelement (M) auf die vorgesehene Reaktionstemperatur vorgeheizt werden. Sie werden in flüssiger Form über Leitung (1) bevorzugt am oberen Ende des Reaktors eingebracht.

Die aus den jeweiligen Reaktoren zu entnehmende Flüssigphase wird am unteren Reaktorende entnommen und über die Leitungen (2) bzw. (3) in die jeweils folgenden Reaktoren (B) oder (C) am oberen Ende wieder eindosiert. Über Leitung (4) wird der Produktstrom entnommen und der Aufarbeitung zugeführt. Die Regelung des gewünschten Füllstandes in den kontinuierlich betriebenen Reaktoren erfolgt auch hier nach bekannten Methoden. Bei der Benutzung einer Kaskade kann die Gasphase durch den kontinuierlich laufenden Flüssigkeitsstrom entweder im Querstrom (Fig. 2) oder im Gegenstrom (Fig. 3) geschickt werden. Querstrom bedeutet hierbei, daß die Reaktionsgase über die Leitungen (12), (13), und (5) (Fig. 2) eindosiert werden und jeweils am oberen Ende eines jeden Reaktors über die Leitungen (8), (7) und (6) (Fig. 2) zusammen mit dem Reaktionswasser und mitgerissenem Edukt (II) wieder entnommen werden, d.h. das Reaktionsgas durchströmt die Reaktoren quer zur Flußrichtung der Flüssigphase. Im Falle einer separaten $O_2$-Dosierung erfolgt dies über die Leitungen (9), (14) und (15). In den Trennorganen (H), (I) und (J) wird das Edukt (II) abgetrennt und in die jeweiligen Reaktoren zurückgeführt. Über die Leitungen (8'), (7') und (6') verläßt das überschüssige Reaktionsgas zusammen mit dem Reaktionswasser den Reaktor.

Das Reaktionswasser wird, nach Vereinigung der Abgasströme, aus dem Reaktionsgas in bekannter Weise entfernt. Das Reaktionsgas wird anschließend zusammen mit dem Ersatz des verbrauchten Reaktionsgases den Reaktoren (A), (B) und (C) wieder zugeführt. Die Gesamtmenge des eindosierten Reaktionsgases kann beliebig auf die einzelnen Reaktoren aufgeteilt werden. In jedem Einzelreaktor wird bevorzugt die Gegenstromfahrweise von Flüssigphase und Gasphase realisiert.

Gegenstromfahrweise (Fig. 3) bedeutet, daß man die Reaktionsgase in den letzten Reaktor (Reaktor C in Fig. 3) über Leitung (5) eindosiert, kontinuierlich gegen die vom ersten Reaktor (A) zum letzten Reaktor (C) in Fig. 3 laufende Flüssigphase durch die Leitungen (6) und (7) führt und am jeweils unteren Ende des folgenden Reaktors (B) und (A) wieder einleitet. Im Falle einer separaten $O_2$-Dosierung erfolgt dies über die Leitungen (9), (14) und (15). Die Dosierung und Führung der Flüssigphase in den Reaktoren ist mit der Querstromfahrweise identisch. Am oberen Ende des ersten Reaktors (A in Fig. 3) wird das überschüssige Reaktionsgas zusammen mit dem Reaktionswasser und mitgerissenem Edukt (II) über Leitung (8) entnommen. Im Trennorgan (H) wird der größte Teil des Edukts (II) abgetrennt und in den Reaktor (A) zurückgeführt. Über die Leitung (8') verläßt das überschüssige Reaktionsgas zusammen mit dem Reaktionswasser den Reaktor. Das Reaktionswasser wird aus dem Reaktionsgas in bekannter Weise entfernt und das Reaktionsgas anschließend zusammen mit dem Ersatz des verbrauchten Reaktionsgases dem Reaktor (C) wieder zugeführt.

Die Aufarbeitung des flüssigen Reaktionsgemisches kann z.B. durch Destillation, Kristallisation oder Extraktion erfolgen. Im Falle einer destillativen Aufarbeitung wird zunächst die nicht umgesetzte aromatische Hydroxyverbindung abgetrennt. In einem weiteren Schritt wird das aromatische Carbonat isoliert. Die im Rückstand befindlichen Katalysatorkomponenten (quaternäres Salz, Base) können zurückgewonnen und recyclisiert werden. Im Falle einer Aufarbeitung durch Kristallisation wird die Reaktionslösung aus dem Reaktor entnommen, in geeigneter Weise abgekühlt und geimpft. In einem ersten Schritt wird dabei ein Kristallisat, bestehend aus einem Gemisch aus Diarylcarbonat und aromatischer Hydroxyverbindung, abgetrennt. Dies kann beispielsweise durch eine fraktionierten Schmelzkristallisation in einem Röhrenkristaller oder durch eine Suspensionskristallisation, beispielsweise in einem Rührkessel, erfolgen. Im Falle einer fraktionierten Schmelzkristallisation werden Nebenprodukte und Reste des Reaktionssystems (quaternäres Salz, Base, aus dem Kristallisat durch Schwitzen abtrennt und gemeinsam mit der Schmelze in den Reaktor zurückgeführt. Im Falle einer Suspensionskristallisation werden Nebenprodukte und Reste des Reaktionssystems (quaternäres Salz, Base) aus dem Kristallisat mit einer wasserfreien Waschlösung, bevorzugt einem Gemisch aus Diarylcarbonat und aromatischer Hydroxyverbindung, herausgewaschen. Schmelze und Waschlösung können anschließend ohne weitere Behandlung in den Reaktor zurückgeführt werden. Die so gereinigten Kristallisate, bestehend aus einem Gemisch aus Diarylcarbonat und aromatischer Hydroxyverbindung, werden verlustfrei durch Kristallisation oder Destillation in hochreines Diarylcarbonat aufgearbeitet, die dabei anfallende aromatische Hydroxyverbindung wird in den Reaktor zurückführt.

Im Falle einer Aufarbeitung durch Extraktion wird die Reaktionslösung aus dem Reaktor entnommen, mit einem selektiven Extraktionsmittel intensiv vermischt und anschließend die Phasen trennt. Die resultierende Abgeberphase ist an aromatischem Carbonat und aromatischer Hydroxyverbindung abgereichert und kann neben den homogenen Reaktionskomponenten (quaternäres Salz, Base) noch einige Prozente gelösten Aufnehmer enthalten. Diese Anteile an gelöstem Aufnehmer können z.B. durch Destillation abgetrennt und in die Extraktionsstufe zurückgegeben werden. Anschließend wird die abgereicherte Abgeberphase in den Reaktor zurückgeführt. Die klaren Aufnehmerphasen enthalten neben dem Extraktionsmittel nur die aromatische Hydroxyverbindung, das aromatische Carbonat sowie geringe Spuren des Katalysatorsystems. Nach Abtrennung (z.B. durch Destillation) und Rückführung des Aufnehmers in die Extraktionsstufe wird der Rückstand durch Kristallisation oder Destillation in hochreines Diarylcarbonat aufgearbeitet, die dabei anfallende aromatische Hydroxyverbindung wird in den Reaktor zurückführt.

**Beispiele:**

**Beispiel 1     Herstellung eines pulverförmigen Trägerkatalysators:**

a) Belegung eines Titandioxid-Pulvers mit Palladium und Mangan:

Zu einer Aufschlämmung von 283,5 g Titandioxid-Pulver (Fa. Norton) in 1 500 ml Wasser wurden bei Raumtemperatur 300 ml Lösung von 40,5 g (0,16 mol) Mangan(II)-nitrat-4-hydrat in Wasser gegeben. Dann wurde mit verdünnter Natronlauge alkalisch gestellt die Suspension wurde abgesaugt, mit Wasser gewaschen, bei 100°C getrocknet und 3 h bei 300°C getempert. Der mit Mangan dotierte Träger wurde in 1 500 ml Wasser aufgeschlämmt und mit 300 ml Lösung, enthaltend 50 g Natriumtetrachloropalladat(II)-Lösung mit 15 % Palladium, versetzt. Dann wurde mit verdünnter Natronlauge alkalisch gestellt. Die Suspension wurde abgesaugt, gewaschen und bei 100°C getrocknet. Der Katalysator enthielt 2,5 % Pd und 3 % Mn, jeweils als Metall gerechnet.

b) Belegung eines Titandioxid-Pulvers mit Palladium und Kobalt:

Zu einer Lösung von 18,75 g Palladium(II)-bromid (0,07 mol), 28,5 g Natriumbromid (0,28 mol) und 33,4 g (Kobalt(II)-bromid (0,15 mol) in 1 500 ml Wasser wurden bei Raumtemperatur 283,5 g Titandioxid-Pulver (Fa. Norton) gegeben. Dann wurde mit verdünnter Natronlauge alkalisch gestellt. Die Suspension wurde abgesaugt, gewaschen und bei 100°C getrocknet. Der Katalysator enthielt 2,5 % Pd und 3 % Co, jeweils als Metall gerechnet.

c) Belegung von Manganoxid-Pulver mit Palladium:

Zu eineer Aufschlämmung von 292,5 g Mangandioxid-Pulver in 1 500 ml Wasser wurden bei Raumtemperatur 300 ml Lösung von 50 g Natriumtetrachloropalladat(II)-Lösung mit 15 % Palladium in Wasser gegeben. Anschließend wurde mit verdünnter Natronlauge alkalisch gestellt. Die Suspension wurde abgesaugt und bei 100°C getrocknet. Der Kontakt enthielt 2,5 % Pd auf $MnO_2$-Träger, gerechnet als Metall.

**Beispiel 2      Herstellung eines stückigen Träger-Katalysators:**

a) Belegung eines Titandioxid-Extrudats mit Palladium und Mangan:

200 ml Titandioxid-Extrudat wurden mit 58,4 ml Lösung von 21,6 g Mangan(II)-chlorid in Wasser getränkt. Dann wurde unter Stickstoff bei 110°C getrocknet. Der mit Mangan dotierte Träger wurde mit 58 ml Lösung, enthaltend 33,3 g Natriumtetrachloropalladat(II)-Lösung mit 15 % Palladium, getränkt. Dann wurde unter Stickstoff bei 110°C getrocknet. Der fertige Kontakt enthielt pro Liter 25 g Pd und 30 g Mn, jeweils als Metall gerechnet

b) Belegung eines Titandioxid-Extrudats mit Rhodium und Mangan:

200 ml Titandioxid-Extrudat wurden mit 58,4 ml Lösung von 21,6 g Mangan(II)-chlorid in Wasser getränkt. Dann wurde unter Stickstoff bei 110°C getrocknet. Der mit Mangan dotierte Träger wurde mit 58 ml Lösung, enthaltend 12,94 g Rhodium(III)-chloridhydrat, getränkt. Dann wurde unter Stickstoff bei 110°C getrocknet. Der Kontakt enthielt pro Liter 25 g Rhodium und 30 g Mn, jeweils als Metall gerechnet.

c) Herstellung von Extrudaten aus pulverförmigen Seltenerdoxidgemisch und Belegung mit Palladium:

Ein kommerziell erhältliches Gemisch aus Seltenerdoxiden (Fa. Rhône-Poulenc) wurde mit Wasser angeteigt, extrudiert, 5 h bei 110°C getrocknet und 5 h bei 400°C calciniert. 200 ml Seltenerdoxid-Extrudat wurden mit 70 ml wäßriger Lösung von 33,3 g Natriumtetrachloropalladat(II)-Lösung mit 15 % Palladium getränkt. Anschließend wurde an Luft bei 110°C getrocknet. Der Kontakt enthielt pro Liter 25 g Pd, gerechnet als Metall.

**Patentansprüche**

1.   Verfahren zur kontinuierlichen Herstellung von Diarylcarbonaten der Formel

$$R^1\text{-O-CO-O-}R^1 \qquad \text{(I)}$$

durch Umsetzung von aromatischen Hydroxyverbindungen der Formel

$$R^1\text{-OH} \qquad \text{(II),}$$

wobei in den Formeln

$R^1$   nicht substituiertes oder substituiertes $C_6$-$C_{15}$-Aryl bedeutet,

mit Kohlenmonoxid und Sauerstoff bei 30-200°C und 1-120 bar in Gegenwart eines Platingruppenmetalls oder einer Verbindung eines Platingruppenmetalls als Katalysator, eines Cokatalysators aus der Gruppe Mn, Cu, Co, V, Zn und Mo, eines quarternären Salzes und einer Base, wobei Katalysator und Cokatalysator gemeinsam als stationär angeordneten oder in fluides Phase vorliegenden. Trägerkatalysator eingesetzt und die Reaktion in der kondensierten Phase durchgeführt wird, die Reaktionslösung kontinuierlich aus dem Reaktor entnommen, gebildetes Diarylcarbonat durch Kristallisation, Destillation oder Extraktion aus der Reaktionslösung abgetrennt, durch weitere Kristallisation oder Destillation weitgehend verlustfrei in hochreines Diarylcarbonat aufgearbeitet und die restliche Reaktionslösung in den Reaktor zurückgeführt wird.

2. Verfahren nach Anspruch 1, **dadurch gekennzeichnet, daß** das Platingruppenmetall des Trägerkatalysators Pt, Ir, Pd, Ru oder Rh oder mehrere von ihnen in einer Menge von 0,01-15 Gew.-%, gerechnet als Metall und bezogen auf das Gesamtgewicht des Katalysators, vorliegt

3. Verfahren nach Anspruch 1, **dadurch gekennzeichnet, daß** als Träger Kohlenstoff, Elementoxide, Elementcarbide oder Elementsalze eingesetzt werden.

4. Verfahren nach Anspruch 5,**dadurch gekennzeichnet, daß** der Cokatalysator in einer Menge von 0,01 - 15 Gew. %, gerechnet als Metall und bezogen auf das Gesamtgewicht des Katalysators vorliegt

5. Verfahren nach Anspruch 1, **dadurch gekennzeichnet, daß** als quaternäres Salz eines oder mehrere aus der Gruppe der Ammonium- und Phosphonium-salze, deren organische Reste gleich oder unterschiedlich sind und $C_6$-$C_{10}$-Aryl, $C_7$-$C_{10}$-Aralkyl und $C_1$-$C_{20}$-Alkyl bedeuten können und die als Anion ein Halogenid, Tetrafluoroborat oder Hexafluorophosphat enthalten, in einer Menge von 0,1-50 Gew.-%, bezogen auf das Gewicht des Reaktionsgemisches, eingesetzt wird.

6. Verfahren nach Anspruch 1, **dadurch gekennzeichnet, daß** als Base ein Hydroxid, Carbonat, $C_2$-$C_{12}$-Carboxylat oder ein Phenolat eines Alkalimetalls, ein tertiäres Amin oder eine Pyridinbase in einer Menge von 0,01-20 Gew.-%, bezogen auf das gesamte Reaktionsgemisch, eingesetzt wird.

7. Verfahren nach Anspruch 1, **dadurch gekennzeichnet, daß** CO und $O_2$ im Molverhältnis von 1: 0,001-1 und in einer Gesamtmenge von 1-100.000 Nl pro 1 Reaktionsgemisch eingesetzt werden.

8. Verfahren nach Anspruch 1, **dadurch gekennzeichnet, daß** der Trägerkatalysator in suspendierter Form in einer Menge von 0, 001-50 Gew.-% bezogen auf die eingesetzte aromatische Hydroxyverbindung, verwendet wird.

9. Verfahren nach Anspruch 1, **dadurch gekennzeichnet, daß** bei 30-150°C und 2-80 bar gearbeitet wird.

**Claims**

1. Process for the continuous production of diaryl carbonates of the formula

$$R^1\text{-O-CO-O-}R^1 \qquad (I)$$

by reacting aromatic hydroxy compounds of the formula

$$R^1\text{-OH} \qquad (II),$$

wherein in the formulae

$R^1$    means unsubstituted or substituted $C_6$-$C_{15}$ aryl,

with carbon monoxide and oxygen at 30-200°C and at 1-120 bar, in the presence of a platinum group metal or a compound of a platinum group metal as the catalyst, a co-catalyst from the group Mn, Cu, Co, V, Zn and Mo, a quaternary salt and a base, wherein catalyst and co-catalyst are used together as a supported catalyst in a stationary arrangement or in the fluid phase and the reaction is performed in the condensed phase, the reaction solution is continuously discharged from the reactor, the diaryl carbonate formed is separated from the reaction solution by crystallisation, distillation or extraction, is worked up substantially without loss by further crystallisation or distillation to yield high purity diaryl carbonate and the remaining reaction solution is returned to the reactor.

2. Process according to claim 1, **characterised in that** the platinum group metal of the supported catalyst Pt, Ir, Pd, Ru or RH or two or more thereof is present in a quantity of 0.01 to 15 wt.% calculated as metal and relative to the entire weight of the catalyst.

3. Process according to claim 1, **characterised in that** carbon, element oxides, element carbides or element salts are used as the support.

4. Process according to claim 5, **characterised in that** the co-catalyst is present in a quantity of 0.01-15 wt.%, calculated as metal and relative to the entire weight of the catalyst.

5. Process according to claim 1, **characterised in that** one or more salts from the group of ammonium and phosphonium salts, the organic residues of which are identical or different and may mean $C_6$ to $C_{10}$ aryl, $C_7$ to $C_{10}$ aralkyl and $C_1$ to $C_{20}$ alkyl and which contain a halide, tetrafluoroborate or hexafluorophosphate as an anion, is (are) used as the quaternary salt in a quantity of 0.1 to 50 wt.%, relative to the weight of the reaction mixture.

6. Process according to claim 1, **characterised in that** a hydroxide, carbonate, $C_2$-$C_{12}$ carboxylate or a phenolate of an alkali metal, a tertiary amine or a pyridine base is used as the base in a quantity of 0.01 to 20 wt.%, relative to the entire reaction mixture.

7. Process according to claim 1, **characterised in that** CO and $O_2$ are used in a molar ratio of 1: 0.001-1 and in a total quantity of 1-100000 Nl per litre of reaction mixture.

8. Process according to claim 1, **characterised in that** the supported catalyst is used in suspended form in a quantity of 0.001 to 50 wt.%, relative to the introduced aromatic hydroxy compound.

9. Process according to claim 1, **characterised in that** it is performed at 30 to 150°C and 2 to 80 bar.

**Revendications**

1. Procédé de préparation en continu de carbonates de diaryle de formule :

$$R^1 - O - CO - O - R^1 \qquad (I)$$

par réaction de composés hydroxylés aromatiques de formule:

$$R^1 - OH \qquad (II)$$

où dans les formules,

$R^1$ représente un radical aryle en $C_6$-$C_{15}$ non substitué ou substitué,

avec du monoxyde de carbone et de l'oxygène à 30-200°C et à 1-120 bar, en présence d'un métal du groupe du platine ou d'un composé d'un métal du groupe du platine comme catalyseur, d'un cocatalyseur choisi parmi le groupe de Mn, Cu, Co, V, Zn et Mo, d'un sel quaternaire et d'une base, où le catalyseur avec le cocatalyseur sont mis en oeuvre comme un catalyseur supporté, disposé de manière stationnaire ou présent en phase fluide et la réaction est réalisée en phase condensée, la solution de réaction est prélevée du réacteur en continu, le carbonate de diaryle formé est séparé de la solution de réaction par cristallisation, distillation ou extraction, celui-ci est traité essentiellement sans perte, en carbonate de diaryle très pur par une autre cristallisation ou distillation et la solution de réaction résiduelle est reconduite dans le réacteur.

2. Procédé suivant la revendication 1, **caractérisé en ce que** le métal du groupe du platine du catalyseur supporté est le Pt, Ir, Pd, Ru ou Rh ou plusieurs de ceux-ci, en une quantité allant de 0,01 à 15% en poids, calculé comme métal et sur base du poids total de catalyseur.

3. Procédé suivant la revendication 1, **caractérisé en ce que** comme support, on met en oeuvre du carbone, un oxyde d'élément, un carbure d'élément ou un sel d'élément.

4. Procédé suivant la revendication 1, **caractérisé en ce que** le cocatalyseur est présent en une quantité allant de 0,01 à 15% en poids, calculé comme métal et sur base du poids total de catalyseur.

5. Procédé suivant la revendication 1, **caractérisé en ce que** comme sel quaternaire, on met en oeuvre un ou plusieurs composés parmi le groupe des sels d'ammonium et de phosphonium, dont les restes organiques sont identiques ou différents et peuvent représenter aryle en $C_6$-$C_{10}$, aralcoyle en $C_7$-$C_{10}$ et alcoyle en $C_1$-$C_{20}$, et qui contiennent comme anion, un halogénure, le tétrafluoroborate ou l'hexafluorophosphate, en une quantité allant de 0,1 à 50% en poids, sur base du poids total du mélange de réaction.

6. Procédé suivant la revendication 1, **caractérisé en ce que** comme base, on met en oeuvre un hydroxyde, carbonate, carboxylate en $C_2$-$C_{12}$ ou un phénolate d'un métal alcalin, une amine tertiaire ou une base pyridine en une quantité allant de 0,01 à 20% en poids, sur base du mélange total de réaction.

7. Procédé suivant la revendication 1, **caractérisé en ce que** l'on met en oeuvre le CO et l'$O_2$ en un rapport molaire de 1:0,001-1 et en une quantité totale de 1-100 000 Nlitres par litre de mélange réactionnel.

8. Procédé suivant la revendication 1, **caractérisé en ce que** l'on utilise le catalyseur supporté sous forme de suspension en une quantité allant de 0,001 à 50% en poids sur base du composé hydroxylé aromatique mis en oeuvre.

9. Procédé suivant la revendication 1, **caractérisé en ce que** l'on travaille à 30-150°C et à 2-80 bar.

Fig. 1

Fig. 2

EP 0 801 051 B1

Fig. 3